# EUROPÄISCHE PATENTANMELDUNG

(11) **EP 4 755 325 A1**
(43) Veröffentlichungstag der Anmeldung: **10.06.2026**
(21) Anmeldenummer: 24217891.1
(22) Anmeldetag: 05.12.2024
(51) Int. Cl.: A61B 17/88, A61C 8/00

(54) **DREHMOMENTSCHLÜSSEL FÜR DEN DENTALBEREICH**

(71) Anmelder: CPM Precision GmbH, 69493 Hirschberg (DE)
(72) Erfinder: Maier, Linus, 68526 Ladenburg (DE)
(74) Vertreter: Graf von Stosch Patentanwaltsgesellschaft mbH

(57) **Zusammenfassung**

Die vorliegende Erfindung betrifft Drehmomentschlüssel für den Dentalbereich, insbesondere in Form einer Dentalratsche, sowie Verfahren zum Herstellen eines solchen Drehmomentschlüssels. Entsprechend wird ein Drehmomentschlüssel (10) für den Dentalbereich vorgeschlagen, umfassend einen Aufnahmekörper (12) zum Bereitstellen einer mechanischen Verbindung zwischen dem Drehmomentschlüssel (10) und einem prothetischen Dentalelement, wobei der Aufnahmekörper (12) eine Rotationsachse (46) des Drehmomentschlüssels (10) definiert, einen ersten Stab (34), welcher sich radial vom Aufnahmekörper (12) erstreckt und mit dem Aufnahmekörper (12) einen Grundkörper bildet, und einen zweiten Stab (14), welcher sich radial vom Aufnahmekörper (12) erstreckt, wobei zumindest ein dem Aufnahmekörper (12) gegenüberliegender längsseitiger Endbereich (20) des zweiten Stabs (14) bei Betätigung in einer ersten Rotationsrichtung relativ zum Grundkörper bewegbar ist. Erfindungsgemäß sind der erste Stab (34) und der zweite Stab (14) monolithisch mit dem Aufnahmekörper (12) gebildet, wobei der zweite Stab (14) eine Aussparung (38) aufweist und wobei der erste Stab (34) im nicht betätigten Zustand des Endbereichs (20) in der Aussparung (38) aufgenommen ist.

## Beschreibung

### Technisches Gebiet

Die vorliegende Erfindung betrifft Drehmomentschlüssel für den Dentalbereich, insbesondere in Form einer Dentalratsche, sowie Verfahren zum Herstellen eines solchen Drehmomentschlüssels.

### Stand der Technik

Im Dentalbereich werden typischerweise Schlüssel eingesetzt, um eine hinreichende Befestigung von beispielsweise Stiften im Kieferknochen zu ermöglichen, beispielsweise in der Mandibula oder Maxilla. Auf diese Weise kann insbesondere im Bereich der Implantologie gewährleistet werden, dass ein Implantat mit einer hinreichenden Verankerung eingesetzt und eine Bewegung des Stifts im Kieferknochen verhindert oder zumindest minimiert werden kann.

Um das Eindrehen eines Stifts zu erleichtern, können Drehmomentschlüssel eingesetzt werden, beispielsweise mit einem Ratschenelement. Auf diese Weise kann das angelegte Drehmoment besser kontrolliert werden. Entsprechend kann die Befestigungskraft oder Schraubenkraft überwacht verhindert werden, dass ein Stift oder eine Schraube überdreht wird und gegebenenfalls den Kieferknochen und die Patientensicherheit gefährdet. Gleichermaßen kann hierdurch gewährleistet werden, dass ein für eine hinreichende Befestigung vorgesehenes Drehmoment für das Eindrehen des Stifts eingehalten wird.

### Darstellung der Erfindung

Erfindungsgemäß wurde erkannt, dass derzeitige Dentalratschen aus mehreren Teilen gefertigt sind, welche vor jeder Verwendung zusammengesetzt werden müssen. Dadurch kann sich die für den jeweiligen medizinischen Eingriff erforderliche Zeit erheblich verlängern, umso mehr, zumal solche Dentalratschen nach der Verwendung wieder demontiert und gründlich gereinigt werden müssen. Die Reinigung solcher mehrteiliger Dentalratschen kann dabei besonders aufwendig sein, um die notwendige Sterilität bereitzustellen.

Darüber hinaus kann das Zusammensetzen der Teile fehleranfällig sein. Es kann dadurch auf Dauer ebenfalls Verschleiß eintreten, wodurch die langfristige strukturelle Stabilität solcher Dentalratschen beeinträchtigt sein kann und/oder ein fehlerhaftes und gegebenenfalls den Patienten gefährdendes Drehmoment zur Befestigung eines Stifts angelegt werden muss.

Es wurde dabei ebenfalls erkannt, dass derzeitige Dentalratschen relativ groß bemessen sind. Insbesondere wurde erkannt, dass eine laterale Erstreckung einer Dentalratsche das korrekte Platzieren der Dentalratsche im vorgesehenen Oralbereich beeinträchtigen kann. Weiterhin kann dadurch der potenzielle Wirkungsbereich für die Drehung der Dentalratsche aufgrund der Anatomie reduziert sein.

Ausgehend von dem bekannten Stand der Technik ist es daher eine Aufgabe der vorliegenden Erfindung, einen Drehmomentschlüssel für den Dentalbereich bereitzustellen, welcher eine einfache Reinigung und Verwendung ermöglicht und bevorzugt platzsparend im Dentalbereich eingesetzt werden kann.

Die Aufgabe wird durch die unabhängigen Ansprüche bzw. die in der Beschreibung genannten Gegenstände gelöst. Vorteilhafte Weiterbildungen ergeben sich aus den Unteransprüchen, der Beschreibung und deren Gegenständen und den Figuren.

Entsprechend wird ein Drehmomentschlüssel für den Dentalbereich vorgeschlagen, umfassend einen Aufnahmekörper zum Bereitstellen einer mechanischen Verbindung zwischen dem Drehmomentschlüssel und einem prothetischen Dentalelement, wobei der Aufnahmekörper eine Rotationsachse des Drehmomentschlüssels definiert, einen ersten Stab, welcher sich radial vom Aufnahmekörper erstreckt und mit dem Aufnahmekörper einen Grundkörper bildet, und einen zweiten Stab, welcher sich radial vom Aufnahmekörper erstreckt, wobei zumindest ein dem Aufnahmekörper gegenüberliegender längsseitiger Endbereich des zweiten Stabs bei Betätigung in einer ersten Rotationsrichtung relativ zum Grundkörper bewegbar ist. Erfindungsgemäß sind der erste Stab und der zweite Stab monolithisch mit dem Aufnahmekörper gebildet und weist der zweite Stab eine Aussparung auf, wobei der erste Stab im nicht betätigten Zustand des Endbereichs in der Aussparung aufgenommen ist.

Dadurch, dass der Aufnahmekörper, der erste Stab und der zweite Stab monolithisch miteinander ausgebildet sind, ist der Drehmomentschlüssel besonders vorteilhaft einteilig ausgebildet und ist folglich keine komplexe Montage vor einem medizinischen beziehungsweise chirurgischen Eingriff notwendig. Ebenfalls kann hierdurch die Reinigung des Drehmomentschlüssels vereinfacht werden, zumal aufgrund der monolithischen Ausgestaltung keine lösbaren, mechanische, Verbindungen, sondern durchgehende und/oder abgeschlossene Strukturen vorgesehen sind. Mit anderen Worten sind keine Verschraubungen, Schnappschlüsse, oder Klemmverbindungen vorgesehen und kann eine einfachere, homogenere Oberflächenstruktur bereitgestellt sein, welche das Reinigen des Drehmomentschlüssels erheblich vereinfacht.

Darüber hinaus wird durch die Aussparung im zweiten Stab und die Aufnahme des ersten Stabs in der Aussparung eine besonders filigrane Ausgestaltung des Drehmomentschlüssels ermöglicht. Denn auf diese Weise wird eine seitliche oder parallele Anordnung des ersten Stabs neben dem zweiten Stab vermieden, sodass die gemeinsame laterale Erstreckung des ersten Stabs und des zweiten Stabs reduziert ist. Der Drehmomentschlüssel gemäß der vorliegenden Erfindung kann somit besonders platzsparend und bevorzugt gewichtssparend ausgebildet sein.

Dies ist insbesondere vorteilhaft im Oralbereich, wo die Dimensionierung des Drehmomentschlüssels durch die lokale Anatomie typischerweise begrenzt ist. Die Aufnahme des ersten Stabs innerhalb des zweiten Stabs ermöglicht entsprechend, dass die laterale Erstreckung des ersten Stabs und des zweiten Stabs bei Betätigung des zweiten Stabs im Vergleich zu herkömmlichen Drehmomentschlüsseln, welche eine Anordnung nebeneinander vorsehen, erheblich reduziert sein kann. Lateral in diesem Sinne ist dabei als eine Richtung zu verstehen, welche sich senkrecht zu den Längsachsen des ersten Stabs und des zweiten Stabs sowie senkrecht zur Rotationsachse erstreckt.

Dadurch, dass der erste Stab in einer Aussparung des zweiten Stabs aufgenommen ist, kann der zweite Stab dennoch stabil mit dem Aufnahmekörper verbunden sein. So kann der zweite Stab, welcher auch als Biegestab zu verstehen ist, an mehreren Stellen des Aufnahmekörpers mit dem Aufnahmekörper verbunden sein, ohne dass dies zu einer mechanischen Begrenzung hinsichtlich des ersten Stabs führt. Dadurch kann sich eine vorteilhafte strukturelle Stabilität ergeben. Beim Betätigen des längsseitigen Endbereichs des zweiten Stabs, kann der zweite Stab sich bevorzugt verbiegen, wobei die Verbindung zum Aufnahmekörper entsprechend nicht beeinträchtigt wird und eine Kraft effizient an den Aufnahmekörper übertragen werden kann, um eine Drehung des Aufnahmekörpers zu bewirken.

Bevorzugt ist der erste Stab in Richtung der Rotationsachse an sich gegenüberliegenden Enden des zweiten Stabs vom zweiten Stab überdeckt. Der erste Stab kann somit an dessen Oberseite und Unterseite vom zweiten Stab überdeckt sein. Der zweite Stab kann dabei derart ausgebildet sein, dass der erste Stab in einer Richtung senkrecht zur Längsrichtung vollständig vom zweiten Stab überdeckt ist. Der erste Stab kann somit im Wesentlichen vollständig innerhalb der vom zweiten Stab definierten Aussparung aufgenommen sein. Dies hat den Vorteil, dass der erste Stab die Betätigung des zweiten Stabs an dessen längsseitigem Endbereich nicht beeinträchtigt und eine besonders filigrane Ausgestaltung des Drehmomentschlüssels bereitgestellt werden kann.

Wie vorstehend beschrieben, wird durch die Aufnahme des ersten Stabs in der Aussparung des zweiten Stabs eine besonders platzsparende Anordnung ermöglicht. Gleichermaßen kann eine versehentliche Betätigung des ersten Stabs durch die Aufnahme in der Aussparung weitestgehend vermieden werden. Eine beabsichtigte Drehmomentübertragung auf den Aufnahmekörper kann somit unterstützt werden.

Darüber hinaus werden durch die Erstreckung des zweiten Stabs sowohl über die (gesamte) Oberseite als auch über die (gesamte) Unterseite des ersten Stabs eine verbesserte mechanische Stabilität des zweiten Stabs und eine potenziell größere Kraftbeaufschlagung und Drehmomentübertragung ermöglicht. Eine größere Dimensionierung in lateraler Richtung kann dabei dennoch vermieden werden. Der erfindungsgemäße Drehmomentschlüssel ist entsprechend im Vergleich zu einer Anordnung, wobei der biegefeste Stab frei liegt, der Biegestab und der biegefeste Stab nebeneinander angeordnet sind oder der Biegestab zumindest teilweise im biegefesten Stab angeordnet ist, besonders vorteilhaft.

Bevorzugt weisen der erste Stab und der zweite Stab zwischen ihren längsseitigen Endbereichen in einer Richtung senkrecht zur Längsrichtung im Wesentlichen die gleiche Erstreckung auf. Die Herstellung und die Handhabung des Drehmomentschlüssels können dadurch weiter vereinfacht werden, zumal entlang der Längsrichtung, welche vom ersten Stab und vom zweiten Stab definiert wird, vorgesehen sein kann, dass keine Vorsprünge aus der Aussparung herausragen. Entsprechend kann eine im Wesentlichen kontinuierliche Kontaktfläche bereitgestellt sein. Weiterhin kann dadurch auch die strukturelle Stabilität des ersten Stabs und des zweiten Stabs optimiert sein.

Die Längsachse des ersten Stabs und die Längsachse des zweiten Stabs sind bevorzugt parallel geführt. Mit anderen Worten können der erste Stab und der zweite Stab sich in einer radialen Ebene erstrecken. Bevorzugt ist der erste Stab dabei vollständig innerhalb der Aussparung angeordnet, wobei beispielsweise lediglich die Verbindungsstelle(n) zwischen dem ersten Stab und dem Aufnahmekörper nicht oder nicht vollständig in dieser Ebene oder parallelen Führung angeordnet ist/sind. Zwischen benachbarten Oberflächen des ersten Stabs und des zweiten Stabs ist dabei bevorzugt ein Spalt vorgesehen, um eine Bewegung des zweiten Stabs relativ zum ersten Stab zu erleichtern.

Bevorzugt sind der erste Stab und der zweite Stabs vollständig voneinander beabstandet. Ein minimaler Abstand zwischen dem ersten Stab und dem zweiten Stab liegt dabei bevorzugt in einem Bereich von 0,1 mm bis 1 mm, in einem Bereich von 0,1 mm bis 0,5 mm oder in einem Bereich von 0,15 bis 0,3 mm. Es können somit ganz geringe Abstände zwischen dem ersten Stab und dem zweiten Stab vorgesehen sein, sodass die Dimensionierung des Drehmomentschlüssel vorteilhaft minimiert werden kann. Solche Abstände können vorteilhaft mittels eines additiven Fertigungsverfahrens ermöglicht werden, wie nachfolgend im Hinblick auf das erfindungsgemäße Verfahren beschrieben wird.

Der erste Stab und der zweite Stab können weiterhin derart konfiguriert sein, dass der zweite Stab bei einer Betätigung des zweiten Stabs in der ersten Rotationsrichtung mit einem vorgegebenen Drehmoment relativ zum ersten Stab bewegbar ist und der erste Stab zumindest teilweise aus der Aussparung herausragt. So kann der zweite Stab beispielsweise zumindest teilweise flexibler ausgestaltet sein, bevorzugt in Richtung des längsseitigen Endbereichs, um ein Verbiegen des zweiten Stabs zu ermöglichen. Durch ein solches Verbiegen, kann die Aussparung nicht mehr vollständig entlang des ersten Stabs ausgerichtet sein, sodass ein entsprechender Bereich des ersten Stabs nicht länger innerhalb der Aussparung aufgenommen ist. Diese Ausgestaltung hat den Vorteil, dass der Drehmomentschlüssel besonders schmal ausgestaltet sein kann und sich eine laterale Erstreckung erst bei einer Betätigung des zweiten Stabs vergrößert.

Bevorzugt liegt das vorgegebene Drehmoment im Bereich zwischen 5 Ncm und 150 Ncm. Insbesondere liegt das vorgegebene Drehmoment im Bereich zwischen 5 Ncm und 30 Ncm oder zwischen 5 Ncm und 15 Ncm. So kann vermieden werden, dass der zweite Stab sich bereits bei geringen Kräften relativ zum ersten Stab bewegt wird und gleichermaßen eine relative Bewegung als Indikator dafür bereitgestellt werden kann, dass eine(r) vorgegebene(n) Befestigungskraft des prothetischen Dentalelements erreicht oder angenähert wird.

Der Drehmomentschlüssel kann vorteilhafterweise aus einem Metall oder einer Metalllegierung gebildet sein. Bevorzugt umfasst das Metall oder die Metalllegierung Titan und/oder Aluminium. Besonders bevorzugt ist der Drehmomentschlüssel aus Ti6Al4V gebildet. Durch die Ausgestaltung aus einem Metall oder Metalllegierung kann eine vorteilhafte strukturelle Festigkeit bereitgestellt sein, wobei dennoch eine vorgegebene Flexibilität gewährleistet ist. Die Verwendung eines Metalls beziehungsweise einer Metalllegierung hat weiterhin den Vorteil, insbesondere gegenüber Kunststoffen, dass bei Verwendung lediglich ein minimaler Verschleiß eintritt und eine Bewegung des zweiten Stabs relativ zum ersten Stab auch bei langfristiger Verwendung noch zuverlässig einem vorgegebenen Kraftaufschlag entspricht. Darüber hinaus kann dadurch eine vereinfachte Reinigung und Sterilisation vorgenommen werden.

Eine Ausgestaltung aus Titan und/oder Aluminium, insbesondere als Legierung, kann weiterhin eine besonders vorteilhafte Zugfestigkeit bereitstellen, wobei das Gesamtgewicht des Drehmomentschlüssels geringgehalten werden kann. Weiterhin wird hierdurch eine dauerhafte Biokompatibilität gewährleistet.

Bevorzugt ist der Drehmomentschlüssel ausschließlich aus einem Metall oder einer Metalllegierung gebildet.

Sowohl der erste Stab als auch der zweite Stab erstrecken sich ausgehend vom Aufnahmekörper und sind damit monolithisch gebildet. Der erste Stab kann dabei über jeweilige Verbindungsstellen mit dem Umfang des Aufnahmekörpers verbunden sein, wobei die Verbindungsstellen in Rotationsrichtung vom zweiten Stab beabstandet sind. Die Verbindungsstellen können somit am Umfang des Aufnahmekörpers vorgesehen sein, bevorzugt in einer Richtung senkrecht zur Rotationsachse. Die Verbindungsstellen sind dabei bevorzugt seitlich vom zweiten Stab angeordnet, liegen somit außerhalb des zweiten Stabs. Die Verbindungsstellen des ersten Stabs sind in Rotationsrichtung von Verbindungsstellen des zweiten Stabs am Umfang des Aufnahmekörpers bevorzugt gleich beabstandet.

Die Anordnung der Verbindungsstellen, beispielsweise zwei Verbindungsstellen, senkrecht zur Rotationsachse und beabstandet zum zweiten Stab hat den Vorteil, dass eine verbesserte Biegefestigkeit des ersten Stabs in Rotationsrichtung bereitgestellt werden kann. Entsprechend kann das Risiko einer versehentlichen Bewegung des ersten Stabs relativ zum Aufnahmekörper bei Betätigung des zweiten Stabs reduziert werden. Weiterhin wird durch diese Anordnung auch eine Bewegung des zweiten Stabs relativ zum ersten Stab erleichtert, zumal die Verbindungsstellen die Bewegung nicht beeinträchtigen. Ebenfalls können durch die Anordnung am Umfang des Aufnahmekörpers weitere Funktionen des Aufnahmekörpers vereinfacht implementiert werden. In dieser Hinsicht ist auch die Aufnahme des ersten Stabs innerhalb der Aussparung des zweiten Stabs besonders vorteilhaft, zumal eine solche Anordnung der Verbindungsstellen bei einer Anordnung des ersten Stabs und des zweiten Stabs nebeneinander nicht möglich ist.

Die Verbindungsstellen des ersten Stabs können dabei in Rotationsrichtung von Verbindungsstellen des zweiten Stabs am Umfang des Aufnahmekörpers gleich beabstandet sein. Alternativ, oder zusätzlich, kann der Aufnahmekörper kreisförmig ausgebildet sein, wobei die Verbindungsstellen des ersten Stabs im Querschnitt des Aufnahmekörpers an sich gegenüberliegenden Seiten des Aufnahmekörpers mit dem Aufnahmekörper verbunden sind. Die gleiche Beabstandung zu den Verbindungsstellen des zweiten Stabs hin hat den Vorteil, dass hierdurch die Biegefestigkeit weiter verbessert werden kann. Weiterhin wird durch die vorteilhafte Anordnung der Verbindungsstellen des ersten Stabs an gegenüberliegenden Seiten des Aufnahmekörpers eine weitere Verbesserung der Kraftverteilung bereitgestellt, zumal der erste Stab über einen größeren Rotationsbereich mit dem Aufnahmekörper verbunden ist.

Die Verbindungsstellen des ersten Stabs sind entsprechend bevorzugt an Stellen des Aufnahmekörpers vorgesehen, welche im Hinblick auf die Rotationsachse einer unterschiedlichen radialen Erstreckungsrichtung entsprechen. Der zweite Stab und die Verbindungsstellen des zweiten Stabs erstrecken sich jedoch bevorzugt innerhalb einer Ebene senkrecht zur Rotationsachse erstrecken. Mit anderen Worten erstreckt sich der zweite Stab zusammen mit seinen Verbindungsstellen bevorzugt innerhalb einer einzigen Ebene. Hierdurch wird eine besonders schmale Ausgestaltung des Drehmomentschlüssels ermöglicht, wobei gleichzeitig eine verbesserte Flexibilität relativ zum Aufnahmekörper bereitgestellt werden kann. Der zweite Stab und der erste Stab können sich somit bevorzugt im Wesentlichen in einer Ebene erstrecken, bis auf Verbindungsstellen des ersten Stabs.

Um für den Drehmomentschlüssel einen Indikator für das angelegte Drehmoment bereitzustellen, können verschiedene Markierungen vorgesehen sein. Bevorzugt weist der erste Stab einen dem Aufnahmekörper gegenüberliegenden längsseitigen Endbereich auf, welcher ein Zeigerelement aufweist. Das Zeigerelement erstreckt sich dabei senkrecht zur Längsrichtung und in Rotationsrichtung und ist dabei für mindestens ein vorgegebenes und auf den zweiten Stab in der ersten Rotationsrichtung einwirkendes Drehmoment kennzeichnend.

Der erste Stab ist im Vergleich zum zweiten Stab im Wesentlichen Biegefest, zumindest bis zu einem vorgegebenen Drehmoment. Mit anderen Worten ist der erste Stab derart konfiguriert, dass eine Bewegung des ersten Stabs relativ zum Aufnahmekörper beim Einwirken eines Drehmoments auf den zweiten Stab nahezu ausgeschlossen ist. Entsprechend kann der erste Stab und dessen Zeigerelement besonders vorteilhaft als Indikator für die eine oder mehrere vorgegebenen Drehmomente dienen. Bevorzugt sind der erste Stab und der zweite Stab derart ausgestaltet, dass, im nicht betätigten Zustand des zweiten Stabs, ausschließlich das Zeigerelement des ersten Stabs aus der Aussparung des zweiten Stabs herausragt.

Das Zeigerelement kann eine Nase umfassen, welche bei einem vorgegebenen maximalen Drehmoment in der ersten Rotationsrichtung mit dem zweiten Stab im Eingriff steht. Der erste Stab ist dabei bevorzugt zumindest bis zum vorgegebenen maximalen Drehmoment im Wesentlichen Biegefest ausgebildet. Die Nase kann somit einen Anschlag bereitstellen, welcher anzeigt, dass ein maximales Drehmoment erreicht ist. Dies kann beispielsweise zum Eindrehen eines Stifts oder eines prothetischen Dentalelements auf eine hinreichende Befestigung hinweisen. Das maximale Drehmoment liegt bevorzugt im Bereich zwischen 5 Ncm und 60 Ncm, besonders bevorzugt zwischen 20 Ncm und 50 Ncm.

Um die Betätigung des zweiten Stabs unabhängig vom ersten Stab weiter zu erleichtern, kann der zweite Stab sich in Längsrichtung über den ersten Stab hinaus erstrecken. Entsprechend kann die Betätigung des zweiten Stabs an dessen längsseitigem Endbereich vereinfacht werden, ohne dass der erste Stab versehentlich berührt wird. Diese Ausgestaltung hat weiterhin den Vorteil, dass das beschriebene, optionale Zeigerelement innerhalb vom zweiten Stab angeordnet und somit bei Betätigung des zweiten Stabs einfach abgelesen werden kann.

Der zweite Stab kann weiterhin einen Steg aufweisen, welcher mit dem ersten Stab bei einer Betätigung des zweiten Stabs in einer zweiten, entgegengesetzten Rotationsrichtung im Eingriff steht. Auf diese Weise kann der erste Stab bei einer Betätigung des zweiten Stabs in der zweiten Rotationsrichtung mitgenommen werden. Dies kann insbesondere für eine Ausgestaltung des Aufnahmekörpers mit einem Ratschenelement vorteilhaft sein, um sicherzustellen, dass der Aufnahmekörper ebenfalls in die zweite Rotationsrichtung bewegt wird.

Der Steg kann beispielsweise an einer längsseitigen Schnittstelle zwischen dem ersten Stab und dem zweiten Stab vorgesehen sein, welche beispielsweise dem längsseitigen Ende des ersten Stabs entspricht.

Um die Betätigung des zweiten Stabs zu erleichtern, kann der zweite Stab an dessen dem Aufnahmekörper gegenüberliegenden längsseitigen Endbereich ein Betätigungselement aufweisen, welches sich in Längsrichtung erstreckt und in Längsrichtung vom ersten Stab beabstandet ist. Das Betätigungselement kann beispielsweise tellerförmig oder scheibenförmig ausgebildet sein und dabei an ihren beiden Seiten jeweils eine Mulde oder einen Hohlraum aufweisen, um einen verbesserten Halt zu ermöglichen. Entsprechend kann das Betätigungselement insbesondere als Halteelement ausgebildet sein. Das Betätigungselement kann das Risiko einer versehentlichen Betätigung des ersten Stabs weiter reduzieren und darüber hinaus auch eine bessere Kennzeichnung für den Benutzer bereitstellen, um die Handhabung weiter zu erleichtern. Durch die Erstreckung des Betätigungselements in Längsrichtung wird weiterhin eine Überlappung mit dem ersten Stab in Längsrichtung verhindert.

Die Aussparung kann gemäß einer Ausführungsform als Öffnung ausgebildet sein. Die Öffnung kann entsprechend einen Durchgang bereitstellen. So kann die Öffnung optional eine beidseitige Bewegung des zweiten Stabs in Rotationsrichtung zu ermöglichen, wobei der erste Stab bei Betätigung des zweiten Stabs entsprechend aus der Öffnung herausragen kann.

Die Öffnung ist derart zu verstehen, dass der erste Stab, welcher in der Aussparung aufgenommen ist, nur an zwei Seiten vom zweiten Stab begrenzt wird, nämlich an dessen Oberseite und Unterseite. Die Öffnung kann sich dabei bereits vom Aufnahmekörper erstrecken, sodass der zweite Stab beispielsweise ausgehend von seinen Verbindungsstellen bis zum dem Aufnahmekörper gegenüberliegenden längsseitigen Endbereich durch zwei durchgehende Wandelemente ausgebildet ist, welche einerseits über den Aufnahmekörper und andererseits an diesem längsseitigen Endbereich miteinander verbunden sind.

Gemäß einer weiteren Ausführungsform kann die Aussparung zwischen den längsseitigen Endbereichen ein seitliches Verstärkungselement aufweisen, welches derart angeordnet ist, dass es bei einer Betätigung des zweiten Stabs in einer zweiten entgegengesetzten Rotationsrichtung mit dem ersten Stab im Eingriff steht. Das Verstärkungselement kann somit als laterales, durchgehendes Wandelement ausgebildet sein, welches die Oberseite mit der Unterseite des zweiten Stabs verbindet. Durch das Verstärkungselement kann insbesondere die strukturelle Stabilität weiter verbessert werden, sodass beispielsweise eine Genauigkeit und Langlebigkeit des Drehmomentschlüssels verbessert werden kann und/oder der Drehmomentschlüssel für höhere Drehmomente ausgelegt sein kann.

Um die Festigkeit weiter zu verbessern, kann der zweite Stab entsprechend zumindest zwischen den längsseitigen Endbereichen als Profil ausgebildet sein. Das Profil kann im Querschnitt bevorzugt U-förmig oder als rechteckiges C-Profil ausgebildet sein. Auf diese Weise können auf den zweiten Stab einwirkende Drehmomente besonders vorteilhaft auf den Aufnahmekörper übertragen werden. Darüber hinaus bietet dies den Vorteil, dass der erste Stab einen entsprechenden (rechteckigen) Querschnitt aufweist und somit mit geringen Toleranzen an der Kontur des Profils angepasst sein kann, wodurch sich die Dimensionierung des Drehmomentschlüssels weiter verringern lässt.

Die Rotation des Aufnahmekörpers wird durch die Betätigung des zweiten Stabs ermöglicht. Dabei ist der zweite Stab bevorzugt derart ausgebildet, dass dieser zumindest teilweise relativ zum Aufnahmekörper durch Verbiegen bewegt werden kann. Der Grad der Verbiegung kann dabei vorteilhafterweise auf eine Einwirkung eines entsprechenden Drehmoments auf den zweiten Stab hinweisen. Um eine effiziente Kraftübertragung auf den Aufnahmekörper zu erzielen und gleichzeitig eine hinreichende Flexibilität für ein vorgegebenes Drehmoment bereitzustellen, kann bevorzugt vorgesehen sein, dass das Flächenträgheitsmoment des zweiten Stabs sich in Richtung des dem Aufnahmekörper gegenüberliegenden längsseitigen Endbereichs des zweiten Stabs in Längsrichtung kontinuierlich reduziert. Bevorzugt liegt das minimale Flächenträgheitsmoment dabei im Bereich von 90 Prozent bis 20 Prozent des maximalen Flächenträgheitsmoments, bevorzugt im Bereich von 50 Prozent bis 30 Prozent. Entsprechend kann die Biegesteifheit im Bereich angrenzend zum Aufnahmekörper im Vergleich zum Bereich am längsseitigen Endbereich erheblich größer sein. Durch das sich reduzierende Flächenträgheitsmoment kann somit vorteilhafterweise eine hinreichende Stabilität für ein vorgegebenes Drehmoment gewährleistet sein. Gleichermaßen kann dadurch aber auch eine leichtere Handhabung oder Betätigung des zweiten Stabs bereitgestellt sein, sodass eine hinreichende Flexibilität am längsseitigen Endbereich bereitgestellt werden kann, um beispielsweise eine Änderung im auf den zweiten Stab einwirkenden Drehmoment anzuzeigen.

Ein weiterer Vorteil besteht darin, dass der zweite Stab aufgrund des sich variierenden Flächenträgheitsmoments auf vorteilhafte Weise auch flexibler als der erste Stab ausgebildet sein kann. Das sich variierende Flächenträgheitsmoment kann besonders vorteilhaft in einer Ausführungsform mit einem seitlichen Verstärkungselement vorgesehen sein, zumal sich aufgrund des Verstärkungselements, beispielsweise in einem Profil, eine größere Fläche ergibt, über die sich das Flächenträgheitsmoment reduzieren lässt.

Der Drehmomentschlüssel kann im Allgemeinen für eine Vielzahl von Anwendungen konfiguriert sein. So kann der Aufnahmekörper beispielsweise derart ausgestaltet sein, dass dieser beispielsweise unmittelbar zum formschlüssigen Aufnehmen eines Schraubenkopfs oder einer entsprechenden Form eines prothetischen Dentalelements ausgebildet ist. So kann der Aufnahmekörper beispielsweise zum Aufnehmen einer Sechskantschraube oder einer ähnlichen Form ausgebildet sein.

Um einen größeren Einsatzbereich zu ermöglichen, ist der Aufnahmekörper jedoch bevorzugt zum Aufnehmen eines Schraubeinsatzes ausgelegt, insbesondere eines Ratschenelements, um somit eine Dentalratsche bereitzustellen.

Entsprechend weist der Aufnahmekörper bevorzugt eine zum Aufnehmen eines Ratschenelements oder Ratscheninstruments ausgebildete Durchgangsöffnung auf, welche im Querschnitt kreisförmig und konzentrisch mit der Rotationsachse ausgebildet ist. Die Durchgangsöffnung ermöglicht somit, dass verschiedene Werkzeuge oder Schraubeinsätze in den Drehmomentschlüssel beziehungsweise in den Aufnahmekörper eingesetzt oder eingeschoben werden können und der Drehmomentschlüssel für eine Vielzahl von Anwendungen, insbesondere als Dentalratsche, geeignet ist.

Die erfindungsgemäße Aufnahme des ersten Stabs in der Aussparung des zweiten Stabs ist bei einer Ausgestaltung des Drehmomentschlüssels als Dentalratsche besonders vorteilhaft. Denn, wie vorstehend beschrieben, kann auf diese Weise die laterale Erstreckung des Drehmomentschlüssels erheblich reduziert werden. Entsprechend kann der Rotationsbereich auch bei einer Betätigung des zweiten Stabs in einer zweiten, entgegengesetzten Rotationsrichtung vergrößert werden, sodass beispielsweise eine Befestigung eines Stifts mit weniger Drehungen des Drehmomentschlüssels erzielt werden kann.

Um die Funktion eines aufgenommenen Ratschenelements zu unterstützen, kann der erste Stab einen Rastfinger aufweisen, welcher sich in die Durchgangsöffnung hinein erstreckt. Der Rastfinger ist dabei dazu eingerichtet, ein aufgenommenes Ratschenelement bei einer Betätigung des zweiten Stabs in der ersten Rotationsrichtung mitzunehmen und bei einer Betätigung des zweiten Stabs in einer zweiten entgegengesetzten Rotationsrichtung relativ zum Ratschenelement zu bewegen.

Der Rastfinger kann entsprechend als Federelement ausgebildet sein, welches relativ zum ersten Stab und zum Aufnahmekörper bewegbar ist, um somit einerseits ein Drehmoment auf den Aufnahmekörper zu übertragen und andererseits eine Entkopplung mit dem Ratschenelement für die Ratschenfunktion zu unterstützen. Der Rastfinger ist dabei bevorzugt monolithisch mit dem ersten Stab ausgebildet und erstreckt sich somit vom ersten Stab. Es kann entsprechend auf einen separaten Rastmechanismus verzichtet werden, wodurch die mechanische Stabilität der Ratschenfunktion erheblich verbessert und die Reinigung des Drehmomentschlüssels erheblich vereinfacht werden können. Denn erfindungsgemäß ist auch der erste Stab monolithische mit dem Aufnahmekörper ausgebildet, sodass mittels des einstückig ausgebildeten Rastfingers ein einzelner, durchgehender Ratschenmechanismus vorgesehen sein kann.

Der erste Stab kann dabei einen Anschlag aufweisen, welcher derart angeordnet ist, dass dieser bei Betätigung des zweiten Stabs in der ersten Rotationsrichtung mit dem Rastfinger im Eingriff steht. Der Anschlag kann beispielsweise entlang der Durchgangsöffnung am Aufnahmekörper angeordnet sein, sodass der Rastfinger besonders platzsparend ausgebildet und angeordnet sein kann. Der Rastfinger kann bei Betätigung des zweiten Stabs in der ersten Rotationsrichtung entsprechend mit einer Nut am Umfang eines aufgenommenen Ratschenelements im Eingriff stehen, wobei der Anschlag verhindert, dass der Rastfinger aufgrund der Drehung aus der Nut herausgedrängt wird. Denn der Anschlag bewirkt bevorzugt, dass der Rastfinger in der zweiten entgegengesetzten Rotationsrichtung begrenzt wird. Entsprechend bewegt sich der Rastfinger entlang der Rotationsrichtung und kann das Ratschenelement über den Kontakt mit der Nut mitnehmen.

Bevorzugt sind der Anschlag und der Rastfinger derart angeordnet, dass der Rastfinger bei Betätigung des zweiten Stabs in der zweiten entgegengesetzten Rotationsrichtung vom Anschlag beabstandet und/oder eine Erstreckung des Rastfingers in die Öffnung reduziert ist. Der Rastfinger kann beispielsweise nicht länger in die Öffnung hineinragen oder vom Umfang eines Aufgenommenen Ratschenelements weggeführt werden. Bei der Betätigung des zweiten Stabs in der zweiten entgegengesetzten Rotationsrichtung kann der Rastfinger beispielsweise in die erste Rotationsrichtung vorgespannt werden und sich von einer Nut am Umfang eines aufgenommenen Ratschenelements lösen, sodass das Ratschenelement nicht mitgenommen wird.

Um das Einkoppeln und Entkoppeln eines Ratschenelements in der Durchgangsöffnung zu erleichtern, kann der erste Stab ein Entriegelungselement aufweisen, welches sich in Rotationsrichtung von dem Rastfinger erstreckt und welches derart angeordnet ist, dass eine Erstreckung des Rastfingers in die Durchgangsöffnung bei Betätigung des Entriegelungselements in der ersten Rotationsrichtung reduziert ist. Beispielsweise kann in Abhängigkeit von der Konfiguration des Ratschenelements ein Einkoppeln und Entkoppeln eines Ratschenelements erschwert sein, wenn sich der Rastfinger beispielsweise nicht einfach aus der Nut oder in die Nut am Umfang des Ratschenelements herausbewegen beziehungsweise einführen lässt. Entsprechend kann das Entriegelungselement beim Einführen und Herausnehmen des Ratschenelements betätigt werden, um zu verhindern, dass der Rastfinger das Ratschenelement in Richtung der Rotationsachse begrenzt oder sogar blockiert. Nach dem Einführen des Ratschenelements kann das Entriegelungselement entsprechend losgelassen werden, sodass der Rastfinger in eine am Umfang des Ratschenelements angeordnete Nut einrastet.

Bevorzugt ist das Entriegelungselement außerhalb vom ersten Stab betätigbar. So kann der Rastfinger sich in einer radialen Ebene der Rotationsachse erstrecken und optional von Verbindungsstellen des zweiten Stabs und/oder des ersten Stabs umgeben sein. Mit anderen Worten kann der Rastfinger innerhalb des ersten Stabs und bevorzugt auch innerhalb des zweiten Stabs angeordnet sein. Das Entriegelungselement kann sich dabei senkrecht zur Längsrichtung des ersten Stabs erstrecken und sich dabei beispielsweise durch eine Öffnung einer Verbindungsstelle des ersten Stabs hindurch erstrecken, sodass das Entriegelungselement an der äußeren Oberfläche des Drehmomentschlüssels betätigbar ist. Auf diese Weise kann eine besonders vorteilhafte kompakte Ausgestaltung bereitgestellt sein. Gleichermaßen wird gewährleistet, dass das Einführen Einkoppeln und Entkoppeln des Ratschenelements auf einfache Weise unterstützt werden kann.

Der als Dentalratsche konfigurierte Drehmomentschlüssel kann ebenfalls das Ratschenelement umfassen. Entsprechend kann der Drehmomentschlüssel ein in der Durchgangsöffnung lösbar aufgenommenes Ratschenelement umfassen, wobei das Ratschenelement am Umfang eine Vielzahl von voneinander gleich beabstandeten und parallel zur Rotationsachse angeordneten Nuten aufweist, welche im aufgenommenen Zustand des Ratschenelements im Aufnahmekörper mit dem Rastfinger in Eingriff bringbar sind.

Jede Nut kann dabei in Richtung der Rotationsachse an den sich gegenüberliegenden Enden zur äußeren Oberfläche des Ratschenelements hin gerundet oder als durchgehend offene Nut ausgebildet sein. Obwohl jede Nut an diesen gegenüberliegenden Enden alternativ eine jeweilige Kante aufweisen kann, hat die gerundete oder offene Ausgestaltung den Vorteil, dass das Einsetzen weiter vereinfacht werden kann, zumal ein Rastfinger des Drehmomentschlüssels leichter in die entsprechende Nut eingeführt werden kann. Eine separate Entriegelung mittels eines Entriegelungselements ist somit optional nicht erforderlich.

Das Ratschenelement kann dabei in Richtung der Rotationsachse an einem Ende optional einen Sprengring aufweisen, um einen verbesserten Halt im Aufnahmekörper bereitzustellen, wobei der Sprengring in eine an der Innenfläche der Durchgangsöffnung umlaufenden Nut hineingeschoben und daraus auch wieder herausgeschoben werden kann.

Gemäß einem weiteren Aspekt der Erfindung wird ein Verfahren zum Herstellen eines Drehmomentschlüssels für den Dentalbereich vorgeschlagen, umfassend das Erstellen von einem Aufnahmekörper zum Bereitstellen einer mechanischen Verbindung zwischen dem Drehmomentschlüssel und einem prothetischen Dentalelement, wobei der Aufnahmekörper eine Rotationsachse des Drehmomentschlüssels definiert;
einem ersten Stab, welcher sich radial vom Aufnahmekörper erstreckt zum Bilden eines Grundkörpers mit dem Aufnahmekörper; und
einem zweiten Stab, welcher sich radial vom Aufnahmekörper erstreckt, wobei der zweite Stab derart gebildet wird, dass zumindest ein dem Aufnahmekörper gegenüberliegender längsseitiger Endbereich des zweiten Stabs bei Betätigung in einer ersten Rotationsrichtung relativ zum Grundkörper bewegbar ist.

Erfindungsgemäß werden der Aufnahmekörper, der erste Stab und der zweite Stab schichtweise mittels eines additiven Fertigungsverfahrens erstellt, sodass der erste Stab und der zweite Stab monolithisch mit dem Aufnahmekörper gebildet werden und wobei der zweite Stab mit einer Aussparung gebildet wird, worin der erste Stab im nicht betätigten Zustand des Endbereichs aufgenommen ist.

Durch das additive Fertigungsverfahren, beispielsweise ein LB-PBF Verfahren (Englisch: "laser beam-power bed fusion") wird entsprechend eine besonders vorteilhafte, monolithische Struktur des Drehmomentschlüssels ermöglicht, wobei dadurch gleichzeitig eine Aussparung vorgesehen werden kann, worin der erste Stab aufgenommen ist. Auf diese Weise kann eine besonders filigrane und einteilige Ausgestaltung bereitgestellt werden, welche sowohl hinsichtlich der Reinigung als auch hinsichtlich der möglichen, geringen Dimensionierung des Drehmomentschlüssels für die Anwendung im klinischen Dentalbereich besonders vorteilhaft ist. Denn es ist keine Montage oder Zusammensetzen aus verschiedenen Teilen erforderlich und durch die Aufnahme des ersten Stabs innerhalb der Aussparung des zweiten Stabs kann die laterale Erstreckung des Drehmomentschlüssels erheblich verringert werden.

Darüber hinaus kann auch der Nachbearbeitungsaufwand, beispielsweise ein Glasperlenstrahlen, ein Trovalieren, ein DLC Beschichten und/oder ein Lasermarkieren, erheblich reduziert werden. Auf Fertigungsverfahren oder Fertigungsschritte wie Fräsen oder Drehen kann vollständig verzichtet werden. Die Herstellungskosten können entsprechend ebenfalls reduziert werden, wobei durch die einteilige Ausgestaltung gleichwohl eine verbesserte und langfristige strukturelle Stabilität ermöglicht wird.

Die Schichten können derart gebildet werden, dass der erste Stab und der zweite Stabs vollständig voneinander beabstandet sind. Ein minimaler Abstand zwischen dem ersten Stab und dem zweiten Stab liegt dabei bevorzugt in einem Bereich von 0,1 mm bis 1 mm, in einem Bereich von 0,1 mm bis 0,5 mm oder in einem Bereich von 0,15 bis 0,3 mm. Solche geringe Abstände ermöglichen eine besonders platzsparende Ausgestaltung des Drehmomentschlüssels, wobei aufgrund des schichtweisen Erstellens eine gewisse Genauigkeit bereitgestellt werden kann, um zu gewährleisten, dass der zweite Stab zumindest teilweise relativ zum ersten Stab bewegbar ist. Die Erfinder haben erkannt, dass solche geringfügige Abstände und eine solche Genauigkeit nicht mittels Fräsen oder Drehen, sondern nur mit dem erfindungsgemäßen additiven Fertigungsverfahren möglich ist.

Bevorzugt wird jede Schicht mit einer Dicke im Bereich von 10 µm bis 100 µm aufgetragen, bevorzugt im Bereich von 10 µm bis 30 µm. Dadurch können einerseits sehr filigrane Strukturen gebildet werden. Andererseits ermöglicht dies ebenfalls, dass Materialspannungen und/oder die mechanischen Eigenschaften variiert werden können, ohne dass dies einer entsprechenden Nachbearbeitung bedarf.

Insbesondere können die Schichten mittels des additiven Fertigungsverfahrens vorteilhafterweise derart aufgetragen werden, dass sich das Flächenträgheitsmoment des zweiten Stabs in Richtung des dem Aufnahmekörper gegenüberliegenden längsseitigen Endbereichs des zweiten Stabs in Längsrichtung kontinuierlich reduziert. Mit anderen Worten kann das Flächenträgheitsmoment sich ausgehend vom Aufnahmekörper entlang der Längsachse verringern. So kann das minimale Flächenträgheitsmoment beispielsweise im Bereich von 90 Prozent bis 20 Prozent des maximalen Flächenträgheitsmoments liegen, bevorzugt im Bereich von 50 Prozent bis 30 Prozent, sodass die Biegesteifheit im Bereich angrenzend zum Aufnahmekörper im Vergleich zum Bereich am gegenüberliegenden längsseitigen Endbereich erheblich größer sein kann. Eine solche mechanische Stabilität bei einer gleichzeitig hinreichenden Flexibilität kann besonders vorteilhaft durch das additive Fertigungsverfahren bereitgestellt sein. Denn durch das Aufschichten der Schichtlagen können beispielsweise unterschiedliche Materialspannungen und/oder eine unterschiedliche Dichte entlang der Längsrichtung vorgesehen sein, wodurch die Flexibilität des zweiten Stabs an einem vorgegebenen Drehmoment angepasst beziehungsweise bereitgestellt sein kann.

Der Aufnahmekörper, der erste Stab und der zweite Stab können aus einem Pulver aus einem Metall oder einer Metalllegierung gebildet sein, bevorzugt aus Ti6Al4V. Das Pulver wird entsprechend mittels des additiven Fertigungsverfahrens aufgeschmolzen, um eine monolithische Struktur bereitzustellen. Bevorzugt kann das Pulver dabei aus Partikeln mit einer Partikelgröße von 10 µm bis 100 µm, bevorzugt von 20 µm bis 70 µm gebildet sein, um eine sehr genaue Dimensionierung zu ermöglichen. Eine Ausgestaltung aus Ti6Al4V hat sich dabei besonders gut geeignet für ein additives Fertigungsverfahren herausgestellt und ermöglicht zudem eine hohe Zugfestigkeit, beispielsweise bis etwa 1000 MPa. Auf schwere Materialien wie Edelstahl kann entsprechend verzichtet werden.

Das Verfahren kann insbesondere zum Herstellen des erfindungsgemäßen und vorstehend beschriebenen Drehmomentschlüssels verwendet werden.

### Kurze Beschreibung der Figuren

Bevorzugte weitere Ausführungsformen der Erfindung werden durch die nachfolgende Beschreibung der Figuren näher erläutert. Es zeigen:
Figur 1 eine Draufsicht eines Drehmomentschlüssels gemäß der Erfindung;
Figur 2 eine Draufsicht des Drehmomentschlüssels gemäß Figur 1 bei einer Betätigung des zweiten Stabs in einer ersten Rotationsrichtung;
Figur 3 eine Seitenansicht eines Drehmomentschlüssels gemäß der Erfindung mit einem V erstärku ngselement;
Figur 4 eine Seitenansicht des Drehmomentschlüssels gemäß Figur 3 aus einer gegenüberliegenden Richtung;
Figur 5 eine seitliche perspektivische Darstellung eines erfindungsgemäßen Drehmomentschlüssels in einer Detailansicht auf Höhe des Aufnahmekörpers;
Figur 6 eine seitliche perspektivische Darstellung eines erfindungsgemäßen Drehmomentschlüssels in einer Detailansicht auf Höhe des längsseitigen Endbereichs des zweiten Stabs; und
Figur 7 eine Detailansicht entlang eines Querschnitts des Aufnahmekörpers mit einem im Aufnahmekörper aufgenommenen Ratschenelement.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen. Auf eine wiederholte Beschreibung dieser Elemente wird teilweise verzichtet, um Wiederholungen zu vermeiden.

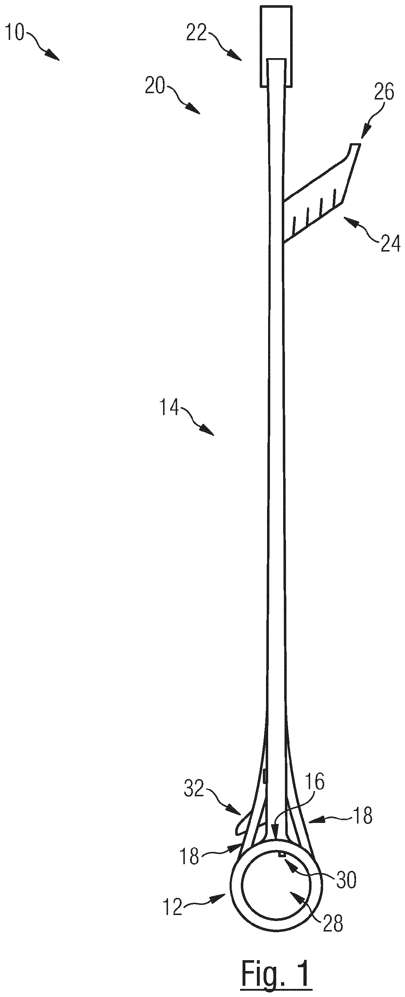

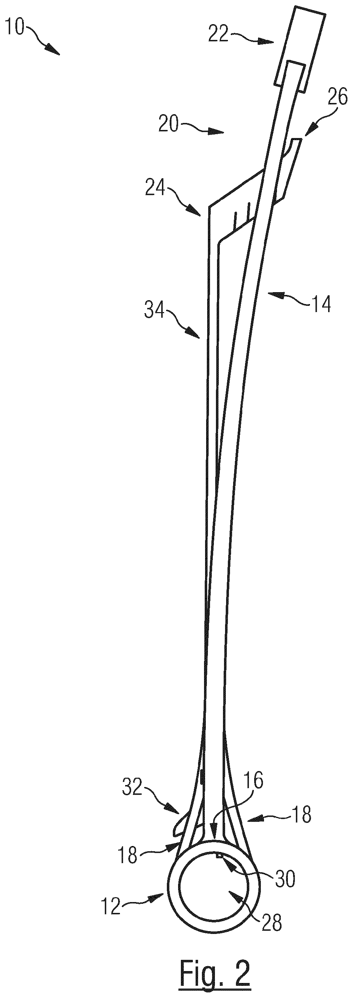

In den Figuren 1 und 2 ist ein Drehmomentschlüssel 10 gemäß der Erfindung in einer Draufsicht gezeigt. Der Drehmomentschlüssel 10 definiert im Wesentlichen eine Längsachse, welche sich radial und im Wesentlichen in einer einzigen Ebene von einem Aufnahmekörper 12 des Drehmomentschlüssels 10 erstreckt. Der Aufnahmekörper 12 hat die Funktion, eine mechanische Verbindung mit einem prothetischen Dentalelement, beispielsweise einem Stift oder einer Schraube, bereitzustellen und somit ein auf den Drehmomentschlüssel 10 einwirkendes Drehmoment auf das prothetische Dentalelement zu übertragen. Um diese Übertragung zu ermöglichen, weist der Drehmomentschlüssel 10 einen ersten Stab 34 sowie einen zweiten Stab 14, welche sich radial vom Aufnahmekörper 12 erstrecken und monolithisch damit gebildet sind.

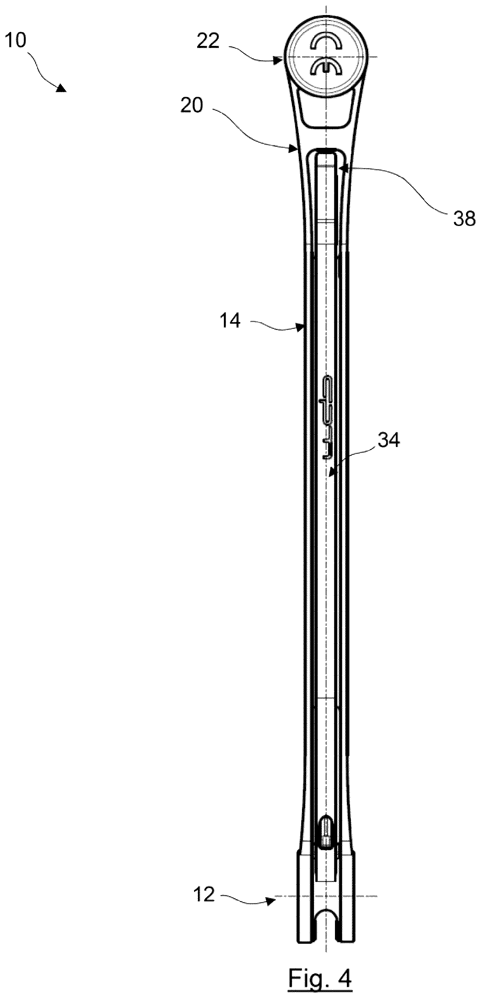

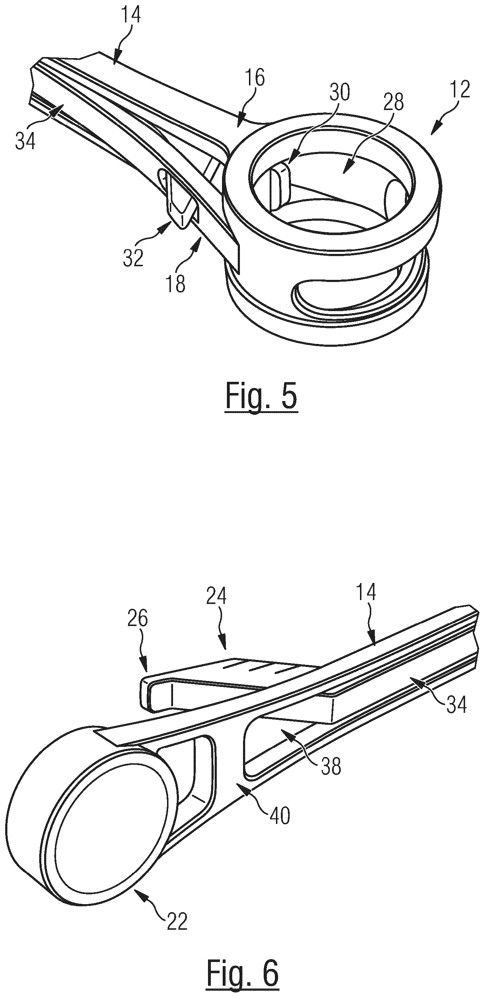

Der erste Stab 34 ist dabei in einer Aussparung 38 des zweiten Stabs 14 aufgenommen. Diese Anordnung ist detaillierter in den Figuren 4 und 6 gezeigt. Entsprechend ist der erste Stab 34 in der Draufsicht gemäß Figur 1 nicht sichtbar, zumal der zweite Stab 14 hier nicht betätigt wird. Um die Betätigung des zweiten Stabs 14 zu erleichtern und dabei zu verhindern, dass der erste Stab 34 versehentlich betätigt wird, weist der zweite Stab 14 an dessen längsseitigem Endbereich 20, welcher dem Aufnahmekörper 12 gegenüberliegt, ein Betätigungselement 22 auf. Das Betätigungselement 22 ist vorliegend tellerförmig ausgebildet.

Bei Betätigung des Betätigungselements 22, bevorzugt mit einem minimalen vorgegebenen Drehmoment, verbiegt sich der zweite Stab 14 zumindest an diesem Endbereich 20. Dadurch ragt der erste Stab 34 zumindest teilweise aus der Aussparung 38 heraus, wie in Figur 2 gezeigt. Um das auf den zweiten Stab 14 einwirkende Drehmoment zu überwachen, weist der erste Stab 14 ein Zeigerelement 24 auf, sodass ein entsprechendes Drehmoment am Zeigerelement 24 ausgelesen werden kann. Wird ein vorgegebenes maximales Drehmoment auf den zweiten Stab 14 angelegt, so kann der zweite Stab 14 mit einer Nase 26 des Zeigerelements 24 im Eingriff stehen. Auf diese Weise kann vorteilhafterweise ein haptisches Feedback eines erzielten Drehmoments bereitgestellt werden.

Der zweite Stab 14 oder zumindest der längsseitiger Endbereich 20 des zweiten Stabs 14 ist dabei im Vergleich zum ersten Stab 34 flexibler ausgebildet. Der erste Stab 34 kann entsprechend im Wesentlichen Biegefest ausgebildet sein. Auf diese Weise wird nicht nur die Validität des Zeigerelements 24 verbessert oder wird das Vorsehen eines Zeigerelements 34 sogar erst ermöglicht. Dies ermöglicht ebenfalls, dass der Drehmomentschlüssel 10 eine vorteilhafte Ratschenfunktion aufweisen kann.

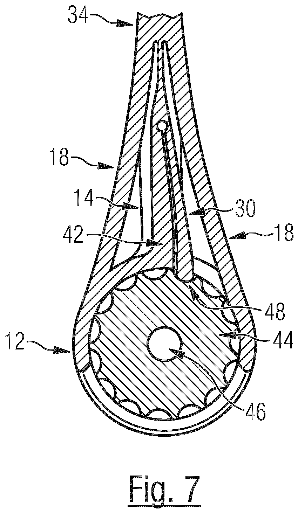

Entsprechend ist im Aufnahmekörper 12 eine Durchgangsöffnung 28 vorgesehen, welcher kreisförmig und konzentrisch mit einer Rotationsachse des Drehmomentschlüssels 10 gebildet ist. Für die Unterstützung einer Ratschenfunktion weist der erste Stab 34 einen Rastfinger 30 auf, welcher in die Durchgangsöffnung 28 radial und in Längsrichtung hineinragt. Der Rastfinger 30 kann dabei mit einem in der Durchgangsöffnung 28 aufgenommenen Ratschenelement (nicht gezeigt) im Eingriff stehen, um das Ratschenelement bei einer ersten Rotationsrichtung des zweiten Stabs 14 mitzunehmen. Der Rastfinger 30 kann optional ein Entriegelungselement 32 aufweisen, welcher sich im Wesentlichen senkrecht oder in Rotationsrichtung vom Rastfinger 30 erstreckt und außerhalb betätigt werden kann, um den Rastfinger 30 in eine Position zu versetzen, welche ein Entkoppeln oder Einkoppeln eines Ratschenelements unterstützt. Diese Funktionen sind im Hinblick auf Figur 7 detaillierter gezeigt und beschrieben.

Für eine verbesserte mechanische Stabilität sind der zweite Stab 14 mittels Verbindungsstellen 16 und der erste Stab 34 mittels Verbindungsstellen 18 mit dem Umfang des Aufnahmekörpers 12 verbunden. Die Verbindungsstellen 18 des ersten Stabs 34 sind dabei vorteilhafterweise vom zweiten Stab 14 und den Verbindungsstellen 16 des zweiten Stabs 14 beabstandet und sind im Querschnitt des Aufnahmekörpers 12 an sich gegenüberliegenden Seiten angeordnet, um eine weitere Biegefestigkeit zu ermöglichen.

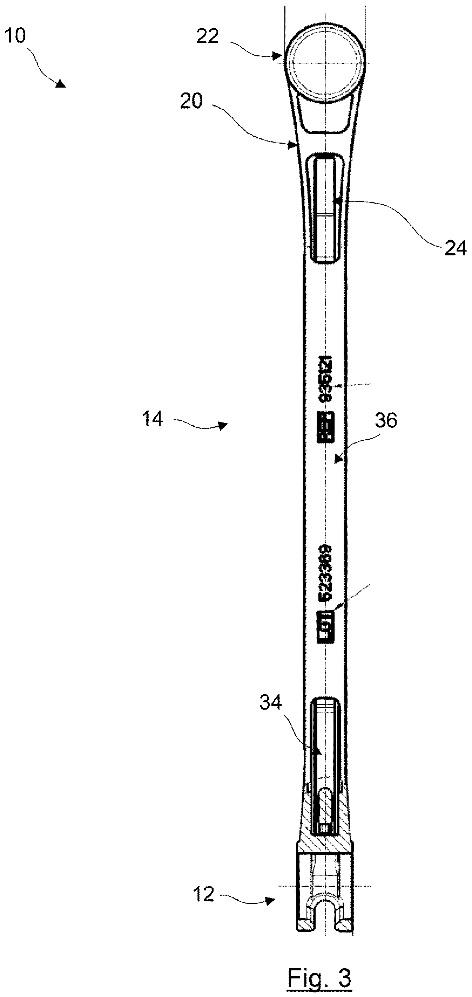

In Figur 3 ist eine Ausgestaltung des Drehmomentschlüssels 10 mit einem seitlichen Verstärkungselement 36 gezeigt. Der zweite Stab 14 definiert folglich weiterhin eine Aussparung 38, ist vorliegend jedoch zumindest zwischen den längsseitigen Endbereichen als optionales Profil ausgebildet. Das seitliche Verstärkungselement 36 bildet somit eine Verbindung zwischen dem oberen Wandelement und unteren Wandelement des zweiten Stabs 14. Der erste Stab 34 ist dabei in der Aussparung 38 aufgenommen, welche in der Figur 3 nicht vollständig sichtbar ist. Dies ist aber daran zu erkennen, dass der erste Stab 34 und das Zeigerelement 24 in den Bereichen, wo das seitliche Verstärkungselement 36 nicht vorgesehen ist, gezeigt sind.

Figur 4 zeigt den Drehmomentschlüssel gemäß Figur 3 aus einer gegenüberliegenden Richtung, wobei die Aussparung 38 und die Anordnung des ersten Stabs 34 innerhalb der Aussparung 38 deutlicher gezeigt sind. Der erste Stab 34 erstreckt sich entsprechend parallel zu den oberen und unteren Wandelementen des zweiten Stabs 14 beziehungsweise der erste Stab 34 und der zweite Stab 14 erstrecken sich im Wesentlichen kollinear ausgehend vom Aufnahmekörper 12 bis zum längsseitigen Endbereich 20 des zweiten Stabs 14. Wie sich aus einer Betrachtung der Figuren 1 bis 4 ergibt, werden durch die erfindungsgemäße Aussparung 38 und Anordnung des ersten Stabs 34 und des zweiten Stabs 14 eine besonders platzsparende Ausgestaltung des Drehmomentschlüssels 10 und aufgrund der filigranen Ausgestaltung eine Anordnung übereinander anstatt nebeneinander ermöglicht.

Figur 5 zeigt eine seitliche perspektivische Darstellung eines erfindungsgemäßen Drehmomentschlüssels 10 in einer Detailansicht auf Höhe des Aufnahmekörpers 12. Zu sehen ist, dass der Rastfinger 30 sowie das Entriegelungselement 32 mit nur geringen Abständen von den benachbarten Oberflächen beabstandet sind und sich durch Strukturen wie die Verbindungsstellen 18 hindurch erstrecken können. Eine solche Ausgestaltung wird vorteilhafterweise durch das erfindungsgemäße schichtweise additive Fertigungsverfahren ermöglicht. Bei Betätigung des Entriegelungselements 32 in Richtung des ersten Stabs 34 kann der Rastfinger 30 entsprechend in eine Richtung weg von der Durchgangsöffnung 28 vorgespannt werden. Hierdurch kann beispielsweise ein Einsetzen oder Herausnehmen eines Ratschenelements vereinfacht werden.

Weiterhin sind vorliegend die Verbindungsstellen 18 des ersten Stabs 34 an sich gegenüberliegenden lateralen Seiten des Aufnahmekörpers 12 vorgesehen, während die Verbindungsstellen 16 des zweiten Stabs 14 oberhalb und unterhalb des ersten Stabs 14 in einer einzigen radialen Ebene vorgesehen sind. Auf diese kann eine mechanische Beeinträchtigung bei einer Rotationsbewegung nahezu vollständig ausgeschlossen und ein potenziell auf den ersten Stab 34 einwirkendes Drehmoment kann erheblich reduziert werden.

In Figur 6 ist eine Detailansicht des Drehmomentschlüssels 10 auf Höhe des längsseitigen Endbereichs 20 des zweiten Stabs 14 in einer seitlichen perspektivischen Ansicht dargestellt. Insbesondere in dieser Figur sind sowohl die Aussparung 38 als auch die Anordnung des ersten Stabs 34 innerhalb der Aussparung 38 gut sichtbar. Durch die Anordnung des ersten Stabs 34 in der Aussparung 38 des zweiten Stabs 14 wird entsprechend eine Überlagerung des ersten Stabs 34 und des zweiten Stabs 14 in Richtung der Rotationsachse beziehungsweise der Vertikale ermöglicht, sodass nicht nur eine besonders filigrane Ausgestaltung ermöglicht, sondern auch eine laterale Erstreckung des Drehmomentschlüssels 10 reduziert werden kann.

In Figur 7 ist eine Detailansicht des Drehmomentschlüssels 10 entlang eines Querschnitts des Aufnahmekörpers 12 mit einem im Aufnahmekörper 12 aufgenommenen Ratschenelement 44 in einem Querschnitt dargestellt. Zu sehen ist hier, dass der Rastfinger 30 sich vom ersten Stab 34 in die Durchgangsöffnung 28 hinein erstreckt. Unmittelbar neben dem Rastfinger 30 befindet sich weiterhin einen Anschlag 42, welcher sich ebenfalls vom ersten Stab 34 erstreckt. Der Anschlag 42 dient zur Begrenzung des Rastfingers 30, sodass die Position des Rastfingers 30 relativ zum aufgenommenen Ratschenelement 44 bei einer Betätigung des zweiten Stabs 14 in der ersten Rotationsrichtung, in der Figur im Uhrzeigersinn, beibehalten bleibt.

Das Ratschenelement 44 ist dabei konzentrisch um die Rotationsachse 46 angeordnet. Entsprechend kann das Ratschenelement 44 beim Anlegen eines Drehmoments am zweiten Stab 14 in der ersten Rotationsrichtung vom Rastfinger 30 mitgenommen werden, um somit eine Drehmomentübertragung auf ein mit dem Ratschenelement 44 gekoppeltes prothetisches Dentalelement bereitzustellen. Der Rastfinger 30 steht dabei im Eingriff mit einem am Umfang des Ratschenelements 44 vorgesehene Nut 48, um eine unmittelbare Übertragung des Drehmoments zu ermöglichen. Der Rastfinger 30 weist dabei jedoch eine gewisse Flexibilität auf. Dadurch wird der Rastfinger 30 bei einer Betätigung des zweiten Stabs 14 in einer zweiten, entgegengesetzten Rotationsrichtung durch die Nut 48 vom Anschlag 42 beabstandet, sodass die Erstreckung des Rastfingers 30 in die Durchgangsöffnung 28 reduziert wird. Aufgrund der reduzierten Erstreckung wird entsprechend eine relative Bewegung des Rastfingers 30 und des Drehmomentschlüssels 10 insgesamt relativ zum Ratschenelement 44 ermöglicht, sodass eine entsprechende Ratschenfunktion bereitgestellt wird.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

Die vorliegende Erfindung wird auch durch die nachfolgenden Gegenstände beschrieben:
1. Drehmomentschlüssel (10) für den Dentalbereich, umfassend
   - einen Aufnahmekörper (12) zum Bereitstellen einer mechanischen Verbindung zwischen dem Drehmomentschlüssel (10) und einem prothetischen Dentalelement, wobei der Aufnahmekörper (12) eine Rotationsachse (46) des Drehmomentschlüssels (10) definiert,
   - einen ersten Stab (34), welcher sich radial vom Aufnahmekörper (12) erstreckt und mit dem Aufnahmekörper (12) einen Grundkörper bildet, und
   - einen zweiten Stab (14), welcher sich radial vom Aufnahmekörper (12) erstreckt, wobei zumindest ein dem Aufnahmekörper (12) gegenüberliegender längsseitiger Endbereich (20) des zweiten Stabs (14) bei Betätigung in einer ersten Rotationsrichtung relativ zum Grundkörper bewegbar ist,
   **dadurch gekennzeichnet, dass**
   der erste Stab (34) und der zweite Stab (14) monolithisch mit dem Aufnahmekörper (12) gebildet sind und der zweite Stab (14) eine Aussparung (38) aufweist, wobei der erste Stab (34) im nicht betätigten Zustand des Endbereichs (20) in der Aussparung (38) aufgenommen ist.
2. Drehmomentschlüssel (10) gemäß Gegenstand 1, wobei der erste Stab (34) in Richtung der Rotationsachse (46) an sich gegenüberliegenden Enden des zweiten Stabs (14) vom zweiten Stab (14) überdeckt ist.
3. Drehmomentschlüssel (10) gemäß Gegenstand 1 oder 2, wobei die Längsachse des ersten Stabs (34) und die Längsachse des zweiten Stabs (14) parallel geführt sind.
4. Drehmomentschlüssel (10) gemäß einem der vorstehenden Gegenstände, wobei der erste Stab (34) und der zweite Stab (14 vollständig voneinander beabstandet sind und ein minimaler Abstand zwischen dem ersten Stab (34) und dem zweiten Stab (14) in einem Bereich von 0,1 mm bis 1 mm, in einem Bereich von 0,1 mm bis 0,5 mm oder in einem Bereich von 0,15 bis 0,3 mm liegt.
5. Drehmomentschlüssel (10) gemäß einem der vorstehenden Gegenstände, wobei der erste Stab (34) und der zweite Stab (14) zwischen ihren längsseitigen Endbereichen in einer Richtung senkrecht zur Längsrichtung im Wesentlichen die gleiche Erstreckung aufweisen.
6. Drehmomentschlüssel (10) gemäß einem der vorstehenden Gegenstände, wobei der erste Stab (34) und der zweite Stab (14) derart konfiguriert sind, dass der zweite Stab (14) bei einer Betätigung des zweiten Stabs (14) in der ersten Rotationsrichtung mit einem vorgegebenen Drehmoment relativ zum ersten Stab (34) bewegbar ist und der erste Stab (34) zumindest teilweise aus der Aussparung (38) herausragt.
7. Drehmomentschlüssel (10) gemäß Gegenstand 6, wobei das vorgegebene Drehmoment im Bereich zwischen 5 Ncm und 150 Ncm liegt.
8. Drehmomentschlüssel (10) gemäß einem der vorstehenden Gegenstände, welcher aus einem Metall oder einer Metalllegierung gebildet ist.
9. Drehmomentschlüssel (10) gemäß Gegenstand 8, wobei das Metall oder die Metalllegierung Titan und/oder Aluminium umfasst oder wobei der Drehmomentschlüssel (10) aus Ti6Al4V gebildet ist.
10. Drehmomentschlüssel (10) gemäß einem der vorstehenden Gegenstände, wobei der erste Stab (34) über jeweilige Verbindungsstellen (18) mit dem Umfang des Aufnahmekörpers (12) verbunden ist, wobei die Verbindungsstellen (18) in Rotationsrichtung vom zweiten Stab (14) beabstandet sind.
11. Drehmomentschlüssel (10) gemäß Gegenstand 10, wobei die Verbindungsstellen (18) des ersten Stabs (34) in Rotationsrichtung von Verbindungsstellen (16) des zweiten Stabs (14) am Umfang des Aufnahmekörpers (12) gleich beabstandet sind.
12. Drehmomentschlüssel (10) gemäß Gegenstand 10 oder 11, wobei der Aufnahmekörper (12) kreisförmig ausgebildet ist und wobei die Verbindungsstellen (18) des ersten Stabs (34) im Querschnitt des Aufnahmekörpers (12) an sich gegenüberliegenden Seiten des Aufnahmekörpers (12) mit dem Aufnahmekörper (12) verbunden sind.
13. Drehmomentschlüssel (10) gemäß einem der vorstehenden Gegenstände, wobei der zweite Stab (14) und die Verbindungsstellen (16) des zweiten Stabs (14) sich innerhalb einer Ebene senkrecht zur Rotationsachse (46) erstrecken.
14. Drehmomentschlüssel (10) gemäß einem der vorstehenden Gegenstände, wobei der erste Stab (14) einen dem Aufnahmekörper (12) gegenüberliegenden längsseitigen Endbereich aufweist, welcher ein Zeigerelement (24) aufweist, welches sich senkrecht zur Längsrichtung und in Rotationsrichtung erstreckt und für mindestens ein vorgegebenes und auf den zweiten Stab (14) in der ersten Rotationsrichtung einwirkendes Drehmoment kennzeichnend ist.
15. Drehmomentschlüssel (10) gemäß Gegenstand 14, wobei das Zeigerelement (24) eine Nase (26) umfasst, welche bei einem vorgegebenen maximalen Drehmoment in der ersten Rotationsrichtung mit dem zweiten Stab (14) im Eingriff steht.
16. Drehmomentschlüssel (10) gemäß Gegenstand 15, wobei der erste Stab (34) zumindest bis zum vorgegebenen maximalen Drehmoment im Wesentlichen Biegefest ausgebildet ist.
17. Drehmomentschlüssel (10) gemäß einem der vorstehenden Gegenstände, wobei der zweite Stab (14) sich in Längsrichtung über den ersten Stab (34) hinaus erstreckt.
18. Drehmomentschlüssel (10) gemäß Gegenstand 17, wobei der zweite Stab (14) einen Steg (40) aufweist, welcher mit dem ersten Stab (34) bei einer Betätigung des zweiten Stabs (14) in einer zweiten entgegengesetzten Rotationsrichtung im Eingriff steht.
19. Drehmomentschlüssel (10) gemäß einem der vorstehenden Gegenstände, wobei die Aussparung (38) als Öffnung ausgebildet ist.
20. Drehmomentschlüssel (10) gemäß einem der Gegenstände 1 bis 18, wobei die Aussparung (38) zwischen den längsseitigen Endbereichen ein seitliches Verstärkungselement (36) aufweist, welches derart angeordnet ist, dass es bei einer Betätigung des zweiten Stabs (14) in einer zweiten entgegengesetzten Rotationsrichtung mit dem ersten Stab (34) im Eingriff steht.
21. Drehmomentschlüssel (10) gemäß Gegenstand 20, wobei der zweite Stab (34) zumindest zwischen den längsseitigen Endbereichen als Profil ausgebildet ist.
22. Drehmomentschlüssel (10) gemäß Gegenstand 21, wobei das Profil im Querschnitt U-förmig oder als rechteckiges C-Profil ausgebildet ist.
23. Drehmomentschlüssel (10) gemäß einem der vorstehenden Gegenstände, wobei das Flächenträgheitsmoment des zweiten Stabs (14) sich in Richtung des dem Aufnahmekörper (12) gegenüberliegenden längsseitigen Endbereichs (20) des zweiten Stabs (14) in Längsrichtung kontinuierlich reduziert.
24. Drehmomentschlüssel (10) gemäß Gegenstand 23, wobei das minimale Flächenträgheitsmoment im Bereich von 90 Prozent bis 20 Prozent des maximalen Flächenträgheitsmoments liegt, bevorzugt im Bereich von 50 Prozent bis 30 Prozent.
25. Drehmomentschlüssel (10) gemäß einem der vorstehenden Gegenstände, wobei der zweite Stab (14) an dessen dem Aufnahmekörper (12) gegenüberliegenden längsseitigen Endbereich (20) ein Betätigungselement (22) aufweist, welches sich in Längsrichtung erstreckt und in Längsrichtung vom ersten Stab (34) beabstandet ist.
26. Drehmomentschlüssel (10) gemäß einem der vorstehenden Gegenstände, wobei der Aufnahmekörper (12) eine zum Aufnehmen eines Ratschenelements (44) ausgebildete Durchgangsöffnung (28) aufweist, welche im Querschnitt kreisförmig und konzentrisch mit der Rotationsachse (46) ausgebildet ist.
27. Drehmomentschlüssel (10) gemäß Gegenstand 26, wobei der erste Stab (34) einen Rastfinger (30) aufweist, welcher sich in die Durchgangsöffnung (28) hinein erstreckt, wobei der Rastfinger (30) dazu eingerichtet ist, das Ratschenelement (44) bei einer Betätigung des zweiten Stabs (14) in der ersten Rotationsrichtung mitzunehmen und bei einer Betätigung des zweiten Stabs (14) in einer zweiten entgegengesetzten Rotationsrichtung relativ zum Ratschenelement (44) zu bewegen.
28. Drehmomentschlüssel (10) gemäß Gegenstand 27, wobei der erste Stab (34) einen Anschlag (42) aufweist, welcher derart angeordnet ist, dass dieser bei Betätigung des zweiten Stabs (14) in der ersten Rotationsrichtung mit dem Rastfinger (30) im Eingriff steht und den Rastfinger (30) in der zweiten entgegengesetzten Rotationsrichtung begrenzt.
29. Drehmomentschlüssel (10) gemäß Gegenstand 28, wobei der Anschlag (42) und der Rastfinger (30) derart angeordnet sind, dass der Rastfinger (30) bei Betätigung des zweiten Stabs (14) in der zweiten entgegengesetzten Rotationsrichtung vom Anschlag (42) beabstandet und/oder eine Erstreckung des Rastfingers (30) in die Durchgangsöffnung (28) reduziert ist.
30. Drehmomentschlüssel (10) gemäß einem der Gegenstände 26 bis 29, wobei der erste Stab (34) ein Entriegelungselement (32) aufweist, welches sich in Rotationsrichtung von dem Rastfinger (30) erstreckt und welches derart angeordnet ist, dass eine Erstreckung des Rastfingers (30) in die Durchgangsöffnung (28) bei Betätigung des Entriegelungselements (32) in der ersten Rotationsrichtung reduziert ist.
31. Drehmomentschlüssel (10) gemäß einem der Gegenstände 27 bis 30, umfassend ein in der Durchgangsöffnung (28) lösbar aufgenommenes Ratschenelement (44), wobei das Ratschenelement (44) am Umfang eine Vielzahl von voneinander gleich beabstandeten und parallel zur Rotationsachse (46) angeordneten Nuten (48) aufweist, welche im aufgenommenen Zustand des Ratschenelements (44) im Aufnahmekörper (12) mit dem Rastfinger (30) in Eingriff bringbar sind und wobei jede Nut (48) in Richtung der Rotationsachse (46) an den sich gegenüberliegenden Enden zur äußeren Oberfläche des Ratschenelements (44) hin gerundet oder als durchgehend offene Nut (48) ausgebildet ist.
32. Verfahren zum Herstellen eines Drehmomentschlüssels (10) für den Dentalbereich, umfassend das Erstellen von
   - einem Aufnahmekörper (12) zum Bereitstellen einer mechanischen Verbindung zwischen dem Drehmomentschlüssel (10) und einem prothetischen Dentalelement, wobei der Aufnahmekörper (12) eine Rotationsachse (46) des Drehmomentschlüssels (10) definiert,
   - einem ersten Stab (34), welcher sich radial vom Aufnahmekörper (12) erstreckt zum Bilden eines Grundkörpers mit dem Aufnahmekörper (12), und
   - einem zweiten Stab (14), welcher sich radial vom Aufnahmekörper (12) erstreckt, wobei der zweite Stab (14) derart gebildet wird, dass zumindest ein dem Aufnahmekörper (12) gegenüberliegender längsseitiger Endbereich (20) des zweiten Stabs (14) bei Betätigung in einer ersten Rotationsrichtung relativ zum Grundkörper bewegbar ist,
   **dadurch gekennzeichnet, dass**
   der Aufnahmekörper (12), der erste Stab (34) und der zweite Stab (14) schichtweise mittels eines additiven Fertigungsverfahrens erstellt werden, sodass der erste Stab (34) und der zweite Stab (14) monolithisch mit dem Aufnahmekörper (12) gebildet werden und wobei der zweite Stab (14) mit einer Aussparung (38) gebildet wird, worin der erste Stab (34) im nicht betätigten Zustand des Endbereichs (20) aufgenommen ist.
33. Verfahren gemäß Gegenstand 32, wobei die Schichten derart gebildet werden, dass der erste Stab (34) und der zweite Stab (14) vollständig voneinander beabstandet sind und ein minimaler Abstand zwischen dem ersten Stab (34) und dem zweiten Stab (14) in einem Bereich von 0,1 mm bis 1 mm, in einem Bereich von 0,1 mm bis 0,5 mm oder in einem Bereich von 0,15 bis 0,3 mm liegt.
34. Verfahren gemäß Gegenstand 32 oder 33, wobei jede Schicht mit einer Dicke im Bereich von 10 µm bis 100 µm aufgetragen wird, bevorzugt im Bereich von 10 µm bis 30 µm.
35. Verfahren gemäß einem der Gegenstände 32 bis 34, wobei die Schichten derart aufgetragen werden, dass sich das Flächenträgheitsmoment des zweiten Stabs (14) in Richtung des dem Aufnahmekörper (12) gegenüberliegenden längsseitigen Endbereichs (20) des zweiten Stabs (14) in Längsrichtung kontinuierlich reduziert.
36. Verfahren gemäß einem der Gegenstände 32 bis 35, wobei der Aufnahmekörper (12), der erste Stab (34) und der zweite Stab (14) aus einem Pulver aus einem Metall oder einer Metalllegierung gebildet sind, bevorzugt aus Ti6Al4V.
37. Verfahren gemäß einem der Gegenstände 32 bis 36 zum Herstellen eines Drehmomentschlüssels (10) gemäß einem der Gegenstände 2 bis 31.

### Bezugszeichenliste

- 10: Drehmomentschlüssel
- 12: Aufnahmekörper
- 14: zweiter Stab
- 16: Verbindungsstelle
- 18: Verbindungsstelle
- 20: längsseitiger Endbereich
- 22: Betätigungselement
- 24: Zeigerelement
- 26: Nase
- 28: Durchgangsöffnung
- 30: Rastfinger
- 32: Entriegelungselement
- 34: erster Stab
- 36: Verstärkungselement
- 38: Aussparung
- 40: Steg
- 42: Anschlag
- 44: Ratschenelement
- 46: Rotationsachse
- 48: Nut

## Patentansprüche

1. Drehmomentschlüssel (10) für den Dentalbereich, umfassend
- einen Aufnahmekörper (12) zum Bereitstellen einer mechanischen Verbindung zwischen dem Drehmomentschlüssel (10) und einem prothetischen Dentalelement, wobei der Aufnahmekörper (12) eine Rotationsachse (46) des Drehmomentschlüssels (10) definiert,
- einen ersten Stab (34), welcher sich radial vom Aufnahmekörper (12) erstreckt und mit dem Aufnahmekörper (12) einen Grundkörper bildet, und
- einen zweiten Stab (14), welcher sich radial vom Aufnahmekörper (12) erstreckt, wobei zumindest ein dem Aufnahmekörper (12) gegenüberliegender längsseitiger Endbereich (20) des zweiten Stabs (14) bei Betätigung in einer ersten Rotationsrichtung relativ zum Grundkörper bewegbar ist,
**dadurch gekennzeichnet, dass**
der erste Stab (34) und der zweite Stab (14) monolithisch mit dem Aufnahmekörper (12) gebildet sind und der zweite Stab (14) eine Aussparung (38) aufweist, wobei der erste Stab (34) im nicht betätigten Zustand des Endbereichs (20) in der Aussparung (38) aufgenommen ist.

2. Drehmomentschlüssel (10) gemäß Anspruch 1, wobei der erste Stab (34) in Richtung der Rotationsachse (46) an sich gegenüberliegenden Enden des zweiten Stabs (14) vom zweiten Stab (14) überdeckt ist und/oder wobei die Längsachse des ersten Stabs (34) und die Längsachse des zweiten Stabs (14) parallel geführt sind.

3. Drehmomentschlüssel (10) gemäß einem der vorstehenden Ansprüche, wobei der erste Stab (34) und der zweite Stab (14) derart konfiguriert sind, dass der zweite Stab (14) bei einer Betätigung des zweiten Stabs (14) in der ersten Rotationsrichtung mit einem vorgegebenen Drehmoment relativ zum ersten Stab (34) bewegbar ist und der erste Stab (34) zumindest teilweise aus der Aussparung (38) herausragt.

4. Drehmomentschlüssel (10) gemäß einem der vorstehenden Ansprüche, welcher aus einem Metall oder einer Metalllegierung gebildet ist, wobei das Metall oder die Metalllegierung bevorzugt Titan und/oder Aluminium umfasst oder wobei der Drehmomentschlüssel (10) bevorzugt aus Ti6Al4V gebildet ist.

5. Drehmomentschlüssel (10) gemäß einem der vorstehenden Ansprüche, wobei der erste Stab (34) über jeweilige Verbindungsstellen (18) mit dem Umfang des Aufnahmekörpers (12) verbunden ist, wobei die Verbindungsstellen (18) in Rotationsrichtung vom zweiten Stab (14) beabstandet sind, wobei die Verbindungsstellen (18) des ersten Stabs (34) bevorzugt in Rotationsrichtung von Verbindungsstellen (16) des zweiten Stabs (14) am Umfang des Aufnahmekörpers (12) gleich beabstandet sind.

6. Drehmomentschlüssel (10) gemäß Anspruch 5, wobei der Aufnahmekörper (12) kreisförmig ausgebildet ist, wobei die Verbindungsstellen (18) des ersten Stabs (34) im Querschnitt des Aufnahmekörpers (12) an sich gegenüberliegenden Seiten des Aufnahmekörpers (12) mit dem Aufnahmekörper (12) verbunden sind und/oder wobei der zweite Stab (14) und die Verbindungsstellen (16) des zweiten Stabs (14) sich innerhalb einer Ebene senkrecht zur Rotationsachse (46) erstrecken.

7. Drehmomentschlüssel (10) gemäß einem der vorstehenden Ansprüche, wobei der erste Stab (14) einen dem Aufnahmekörper (12) gegenüberliegenden längsseitigen Endbereich aufweist, welcher ein Zeigerelement (24) aufweist, welches sich senkrecht zur Längsrichtung und in Rotationsrichtung erstreckt und für mindestens ein vorgegebenes und auf den zweiten Stab (14) in der ersten Rotationsrichtung einwirkendes Drehmoment kennzeichnend ist.

8. Drehmomentschlüssel (10) gemäß Anspruch 7, wobei das Zeigerelement (24) eine Nase (26) umfasst, welche bei einem vorgegebenen maximalen Drehmoment in der ersten Rotationsrichtung mit dem zweiten Stab (14) im Eingriff steht, wobei der erste Stab (34) bevorzugt zumindest bis zum vorgegebenen maximalen Drehmoment im Wesentlichen Biegefest ausgebildet ist.

9. Drehmomentschlüssel (10) gemäß einem der vorstehenden Ansprüche, wobei die Aussparung (38) als Öffnung ausgebildet ist oder wobei die Aussparung (38) zwischen den längsseitigen Endbereichen ein seitliches Verstärkungselement (36) aufweist, welches derart angeordnet ist, dass es bei einer Betätigung des zweiten Stabs (14) in einer zweiten entgegengesetzten Rotationsrichtung mit dem ersten Stab (34) im Eingriff steht.

10. Drehmomentschlüssel (10) gemäß Anspruch 9, wobei der zweite Stab (34) zumindest zwischen den längsseitigen Endbereichen als Profil ausgebildet ist, wobei das Profil im Querschnitt bevorzugt U-förmig oder als rechteckiges C-Profil ausgebildet ist.

11. Drehmomentschlüssel (10) gemäß einem der vorstehenden Ansprüche, wobei das Flächenträgheitsmoment des zweiten Stabs (14) sich in Richtung des dem Aufnahmekörper (12) gegenüberliegenden längsseitigen Endbereichs (20) des zweiten Stabs (14) in Längsrichtung kontinuierlich reduziert, wobei das minimale Flächenträgheitsmoment bevorzugt im Bereich von 90 Prozent bis 20 Prozent des maximalen Flächenträgheitsmoments liegt, besonders bevorzugt im Bereich von 50 Prozent bis 30 Prozent.

12. Drehmomentschlüssel (10) gemäß einem der vorstehenden Ansprüche, wobei der Aufnahmekörper (12) eine zum Aufnehmen eines Ratschenelements (44) ausgebildete Durchgangsöffnung (28) aufweist, welche im Querschnitt kreisförmig und konzentrisch mit der Rotationsachse (46) ausgebildet ist, wobei der erste Stab (34) einen Rastfinger (30) aufweist, welcher sich in die Durchgangsöffnung (28) hinein erstreckt, wobei der Rastfinger (30) dazu eingerichtet ist, das Ratschenelement (44) bei einer Betätigung des zweiten Stabs (14) in der ersten Rotationsrichtung mitzunehmen und bei einer Betätigung des zweiten Stabs (14) in einer zweiten entgegengesetzten Rotationsrichtung relativ zum Ratschenelement (44) zu bewegen.

13. Drehmomentschlüssel (10) gemäß Anspruch 12, wobei der erste Stab (34) ein Entriegelungselement (32) aufweist, welches sich in Rotationsrichtung von dem Rastfinger (30) erstreckt und welches derart angeordnet ist, dass eine Erstreckung des Rastfingers (30) in die Durchgangsöffnung (28) bei Betätigung des Entriegelungselements (32) in der ersten Rotationsrichtung reduziert ist.

14. Verfahren zum Herstellen eines Drehmomentschlüssels (10) für den Dentalbereich, bevorzugt eines Drehmomentschlüssels (10) gemäß einem der vorstehenden Ansprüche, umfassend das Erstellen von
- einem Aufnahmekörper (12) zum Bereitstellen einer mechanischen Verbindung zwischen dem Drehmomentschlüssel (10) und einem prothetischen Dentalelement, wobei der Aufnahmekörper (12) eine Rotationsachse (46) des Drehmomentschlüssels (10) definiert,
- einem ersten Stab (34), welcher sich radial vom Aufnahmekörper (12) erstreckt zum Bilden eines Grundkörpers mit dem Aufnahmekörper (12), und
- einem zweiten Stab (14), welcher sich radial vom Aufnahmekörper (12) erstreckt, wobei der zweite Stab (14) derart gebildet wird, dass zumindest ein dem Aufnahmekörper (12) gegenüberliegender längsseitiger Endbereich (20) des zweiten Stabs (14) bei Betätigung in einer ersten Rotationsrichtung relativ zum Grundkörper bewegbar ist,
**dadurch gekennzeichnet, dass**
der Aufnahmekörper (12), der erste Stab (34) und der zweite Stab (14) schichtweise mittels eines additiven Fertigungsverfahrens erstellt werden, sodass der erste Stab (34) und der zweite Stab (14) monolithisch mit dem Aufnahmekörper (12) gebildet werden und wobei der zweite Stab (14) mit einer Aussparung (38) gebildet wird, worin der erste Stab (34) im nicht betätigten Zustand des Endbereichs (20) aufgenommen ist.

15. Verfahren gemäß Anspruch 14, wobei die Schichten derart aufgetragen werden, dass sich das Flächenträgheitsmoment des zweiten Stabs (14) in Richtung des dem Aufnahmekörper (12) gegenüberliegenden längsseitigen Endbereichs (20) des zweiten Stabs (14) in Längsrichtung kontinuierlich reduziert
